(19) Europäisches Patentamt
European Patent Office
Office européen des brevets

(11) Publication number: **0 083 158**
**B1**

(12) **EUROPEAN PATENT SPECIFICATION**

(45) Date of publication of patent specification: **07.08.85**

(51) Int. Cl.$^4$: **C 07 C 62/16,** C 07 C 51/373, B 01 J 31/28

(21) Application number: **82306401.9**

(22) Date of filing: **02.12.82**

(54) **Novel aldehydes and process for producing the same.**

(30) Priority: **10.12.81 JP 197679/81**

(43) Date of publication of application: **06.07.83 Bulletin 83/27**

(45) Publication of the grant of the patent: **07.08.85 Bulletin 85/32**

(84) Designated Contracting States: **DE FR GB NL SE**

(58) References cited:
**EP-A-0 011 272**
**EP-A-0 014 796**
**GB-A-1 448 812**
**US-A-2 894 038**

(73) Proprietor: **TOA NENRYO KOGYO KABUSHIKI KAISHA**
**1-1 Hitotsubashi, 1-Chome Chiyoda-Ku**
**Tokyo 100 (JP)**

(72) Inventor: **Sakakibara, Tadamori**
**1902-5, Ooaza Kamekubo Ooi-machi**
**Iruma-gun Saitama-ken (JP)**
Inventor: **Wada, Shozo**
**7-5, Yamanone 2-chome**
***Zushi-shi Kanagawa-ken* (JP)**
Inventor: **Kaneko, Katsumi**
**2174-19, Ooaza Nishitsurugaoka**
**Ooi-Machi Iruma-gun Saitama-ken (JP)**

(74) Representative: **Dew, Melvyn John et al**
**Esso Chemical Ltd. Esso Chemical Research Centre P.O. Box 1**
**Abingdon Oxfordshire, OX13 6BB (GB)**

**EP 0 083 158 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel aldehydes and to a process for producing the same. More particularly, the present invention relates to 4-formylcyclohexane-1,2-dicarboxylic acid and to certain substituents and derivatives thereof.

The new aldehydes of this invention are useful as intermediates for hexahydrotrimellitic acid and derivatives thereof and trimellitic acid and derivatives thereof which are useful as plasticizer or raw material of heat resistant plastics.

In accordance with one aspect of the invention there is provided novel aldehydes represented by the formula:

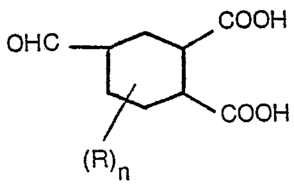

(I)

where R is an alkyl group, alkoxyl group, hydroxyl group, or halogen atom, R's as many as n being the same or different, and n is a number from 0 to 4, or a derivative thereof selected from (mono, di)ester, (mono, di)acid halide, acid anhydride, (mono, di)acid amide and (mono, di)nitrile.

In the above formula (I), the alkyl group represented by R includes lower alkyl groups having 1 to 4 carbon atoms such as methyl, ethyl, propyl, isopropyl, butyl, isobutyl and t-butyl. The alkoxyl group includes, for example, lower alkoxyl groups having 1 to 4 carbon atoms such as methoxy, ethoxy, propoxy, isopropoxy, butoxy, isobutoxy, and t-butoxy. The halogen atom includes chlorine, bromine and iodine atom.

According to another aspect of the invention the compound (I) is produced by reacting a 4-cyclohexene-1,2-dicarboxylic acid represented by the formula (II):

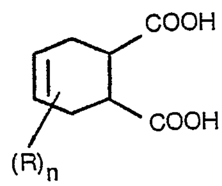

(II)

or a derivative thereof selected from (mono, di)ester, (mono, di)acid halide, acid anhydride, (mono, di)acid amide, and (mono, di)-nitrile, where R and n are defined as above, with hydrogen and carbon monoxide in the presence of a hydroformylation catalyst.

The hydroformylation catalyst used for reacting the above-mentioned compound (II) or its derivative with hydrogen and carbon monoxide includes catalysts containing rhodium, cobalt or platinum. Examples of such catalysts are described below.

By way of example the hydroformylation catalyst containing rhodium (rhodium catalyst) may be rhodium, carbonyl or rhodium carbonyl complex compound with a ligand compound (described below) added thereto, or any one which forms these compounds in the reaction system. Examples of such rhodium catalysts are rhodium black, supported metallic rhodium, rhodium oxide, rhodium nitrate, rhodium chloride, rhodium bromide, rhodium iodide, rhodium acetate, rhodium octylate, $Rh(CO)_2$ (acac), RhCl (CO) $(PPh_3)_2$, RhCl $(PPh_3)_3$, $[RhCl (CO)_2]_2$, HRh (CO) $(PPh_3)_3$, HRh(CO) $(AsPh_3)_3$, and HRh(CO) $[P(OPh)_3]_3$ (where acac stands for acetylacetonate and Ph stands for phenyl).

The rhodium catalyst may be used in combination with an unidentate ligand compound represented by the formula $Z(R^1)(R^2)(R^3)$ where Z denotes a phosphorus, arsenic, or antimony atom, and $R^1$, $R^2$, and $R^3$ denote the same or different alkyl, alkoxyl, aryl, or aryloxy groups; or a bidentate ligand compound represented by the formula $(R^4)_2Z'—D—A—D—Z'(R^5)_2$ where Z' denotes phosphorus, arsenic, or antimony atom, D denotes methylene or a substituted methylene or oxygen atom, A denotes alkylene, cycloalkylene, arylene, or aralkylene having 1 to 10 carbon atoms, $R^4$ and $R^5$ are the same or different aralkyl, aryl, alkoxyl, or aryloxy groups.

The phosphorus compound among the above-mentioned unidentate ligand compounds includes, for example, tertiary phosphine compounds such as $PEt_3$, $PBu_3$, and $PPh_3$; tertiary phosphite compounds such as $P(OEt)_3$, $P(OBu)_3$, and $P(OPh)_3$; tertiary phosphinite compounds such as $Et_2P(OMe)$, $Bu_2P(OEt)$, and $Ph_2P(OPh)$; and tertiary phosphonite compounds such as $PhP(OMe)_2$, $BuP(OEt)_2$, and $PhP(OPh)_2$. The arsenic compound includes, for example, tertiary arsine compounds such as $AsMe_3$, $AsEt_3$, and $AsPh_3$; and arsenious acid ester compounds such as $As(OEt)_3$, $As(OBu)_3$, and $As(OPh)_3$. The antimony compound includes, for example, tertiary stibine compounds such as $SbEt_3$, $SbBu_3$, and $SbPh_3$. Preferred among them are tertiary phosphine compounds and tertiary arsine compounds. (Me, Et, Bu, and Ph denote methyl, ethyl, butyl, and phenyl, respectively).

The above-mentioned bidentate compound includes, for example, 1,3-bis(diphenylphosphino)propane $Ph_2P(CH_2)_3PPh_2$, 1,4-bis(diphenylphosphino)butane $Ph_2P(CH_2)_4PPh_2$, 1,5-bis(diphenylphosphino)pentane $Ph_2P(CH_2)_5PPh_2$, 1,2-bis(diphenylphosphinomethyl)cyclobutane

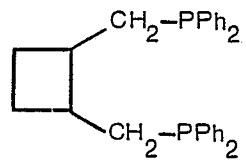

1,2-bis(diphenylphosphinoxy)cyclopentane

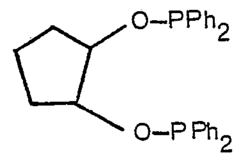

1,2-bis(diphenylphosphinomethyl)cyclopentane

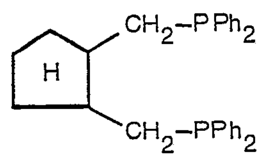

1,2-bis(diphenylphosphinomethyl)cyclohexane

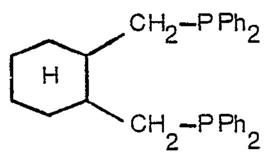

1,3-bis(diphenylphosphinoxy)cyclohexane

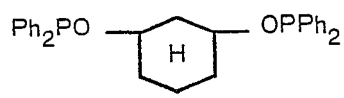

1,2-bis(diphenylphosphinomethyl)benzene

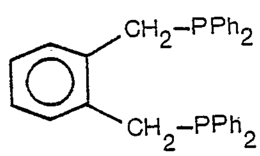

1,3-bis(diphenylphosphinoxy)benzene

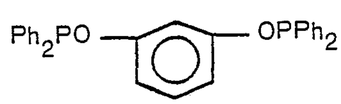

1,8-bis(diphenylphosphinomethyl)naphthalene

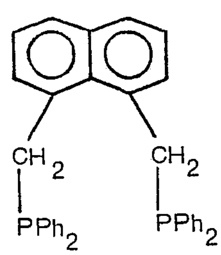

1,2-bis(diphenylarsinomethyl)cyclobutane, 2,3-o-isopropylidene 2,3-dihydroxy-1,4-bis(diphenylarsino)-butane, and arsenic and antimony analogs thereof.

The above-mentioned ligand compound is preferably used in combination with the rhodium catalyst in an amount of 0.1 to 300 mol, more preferably 2 to 50 mol, for 1 mol of the rhodium catalyst.

The catalyst containing platinum (platinum catalyst) may be any one which forms in the reaction system a platinum carbonyl complex with a ligand compound (described above) added thereto. Examples of such platinum catalysts are metallic platinum supported on activated carbon; inorganic salts such as platinum chloride and $K_2PtCl_4$; $Pt(PhCN)_2Cl_2$, $Pt(1,5\text{-}COD)Cl_2$; complex compounds with aforesaid ligand compound such as $Pt(PPh_3)_2Cl_2$ and $Pt(Ph_2PCH_2CH_2PPh_2)Cl_2$ where Ph and COD stand for phenyl and cyclooctadiene, respectively.

If any one of the above-mentioned platinum catalysts which is not a complex compound with a ligand compound is used, the catalyst is preferably used in combination with the above mentioned ligand compound.

Also, the platinum catalyst (and ligand compound) are preferably used together with a co-catalyst which is a halide of an element of Group IVA of the periodic table, (published by the Fisher Scientific Company) such as tin chloride, tin bromide, tin iodide, germanium chloride, and lead chloride.

The platinum catalyst, ligand compound, and co-catalyst are preferably used in the molar ratio of 1/0.1—5/1—20, more preferably 1/0.5—2/2—8.

The catalyst containing cobalt (cobalt catalyst) may be cobalt carbonyl or hydrocobalt carbonyl, or any one which forms these compounds in the reaction system. Examples of such cobalt catalysts are metallic cobalt, oxides ($Co_2O_3$, CoO), salts of inorganic acids ($CoCl_2$, $CoBr_2$, $Co(NO_3)_2$, $CoCO_3$), and salts of organic acids (cobalt acetate, cobalt acetylacetonate).

In a preferred embodiment the reaction in the presence of the above-mentioned hydroformylation catalyst is carried out under the following conditions.

Where a rhodium catalyst is used: reaction pressure is $9.8 \times 10^5$ to $2.94 \times 10^7$Pa (10 to 300 kg/cm$^2$), preferably $1.47 \times 10^6$ to $9.8 \times 10^6$Pa (15 to 100 kg/cm$^2$); reaction temperature is 30 to 150°C, preferably 50 to 120°C; hydrogen/carbon monoxide (molar ratio) is 1/10—10/1, preferably 1/3 to 5/1. Where a platinum catalyst is used: reaction pressure is $9.8 \times 10^5$ to $1.96 \times 10^7$Pa (10 to 200 kg/cm$^2$), preferably $2.94 \times 10^6$ to $9.8 \times 10^6$Pa (30 to 100 kg/cm$^2$); reaction temperature is 30 to 150°C, preferably 50 to 100°C; hydrogen/carbon monoxide (molar ratio) is 1/10—10/1, preferably 1/3 to 5/1. Where a cobalt catalyst is used: reaction pressure is $9.8 \times 10^6$ to $2.94 \times 10^7$Pa (100 to 300 kg/cm$^2$), preferably $1.96 \times 10^7$ to $2.94 \times 10^7$Pa (200 to 300 kg/cm$^2$); reaction temperature is 100 to 200°C, preferably 130 to 160°C; hydrogen/carbon monoxide (molar ratio) is 1/10—10/1, preferably 1/3 to 5/1.

The hydroformylation reaction may be carried out in a solvent which does not adversely affect the reaction, for example, aromatic hydrocarbons such as benzene, toluene, and xylene; saturated aliphatic hydrocarbons such as hexane, heptane, octane, isooctane, and decane; ketones such as acetone, methyl ethyl ketone, methyl isobutyl ketone, acetophenone, and cyclohexanone; and ethers such as tetrahydrofuran, dimethoxyethane, and dioxane.

The invention will now be described in detail with reference to the following examples.

## Example 1

In a 200-ml stainless steel autoclave equipped with a stirrer were placed under a nitrogen atmosphere 15.2 g (100 mmol) of 4-cyclohexene-1,2-dicarboxylic acid anhydride, 0.184 g (0.2 mmol) of carbonyl tris(triphenylphosphine) rhodium hydride [$HRh(CO)(PPh_3)_3$] as a catalyst, and 100 ml of toluene as a solvent. The autoclave was heated, and when the contents reached 115°C, synthesis gas (molar ratio of $H_2$/CO=1) was introduced at $1.96 \times 10^6$Pa (20 kg/cm$^2$). Reaction was carried out with stirring, while keeping the reaction pressure at $1.96 \times 10^6$Pa (20 kg/cm$^2$) with synthesis gas. After 2 hours of reaction, the autoclave was cooled rapidly.

The reaction product removed from the autoclave was freed of low-boiling materials and then distilled in vacuo. 5.94 g of the first fraction (b.p. = 150 to 160°C/0.05 mmHg), which was colorless, transparent, and viscous, and 11.14 g of the second fraction (b.p. = 160—165°C/0.05 mmHg) were obtained.

The second fraction was found to be composed of a single component according to gas chromatography and was identified as 4-formylcyclohexane-1,2-dicarboxylic acid anhydride according to the IR spectrum analysis, NMR spectrum analysis, and elemental analysis which are described below.

IR spectrum analysis (toluene solution)

$\nu_{C=O}$ of acid anhydride: 1860 cm$^{-1}$ (weak), 1790 cm$^{-1}$ (strong)

$\nu_{C=O}$ of aldehyde: 1725 cm$^{-1}$ (medium)

$\nu_{C-H}$ of aldehyde: 2820 cm$^{-1}$ (weak), 2720 cm$^{-1}$ (weak)

NMR spectrum analysis

($CD_3\overset{\underset{\|}{O}}{C}CD_3$ solution, TMS internal reference)

δ value 9.6 ppm (doublet, 1H, proton of $H^a$)
δ value 3.5 ppm (multiplet, 2H, proton of $H^b$)
δ value 2.1 ppm (multiplet, 7H, proton of $H^c$)

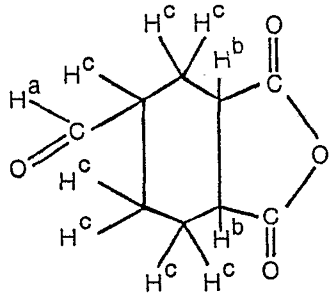

Elemental analysis (as $C_9H_{10}O_4$)
Found (%): C 59.54, H 5.70
Calculated (%): C 59.33, H 5.53.

On the other hand, the first fraction was found by gas chromatography to be composed of cyclohexane-1,2-dicarboxylic acid anhydride (hydrogenation product of raw material), 1-cyclohexene-1,2-dicarboxylic acid anhydride (isomerization product of raw material), 4-cyclohexene-1,2-dicarboxylic acid anhydride (raw material), and 4-formylcyclohexane-1,2-dicarboxylic acid anhydride.

Example 2

The hydroformylation reaction was performed on 4-cyclohexene-1,2-dicarboxylic acid anhydride in the same manner as in Example 1, except that the catalyst was replaced by 0.184 g (0.2 mmol) of HRh(CO)$(PPh_3)_3$ and 0.525 g (2.0 mmol) of triphenylphosphine ($PPh_3$). The reaction product was analyzed by gas chromatography. The results are shown in the table below.

Example 3

The hydroformylation reaction was performed on 4-cyclohexene-1,2-dicarboxylic acid anhydride in the same manner as in Example 1, except that the catalyst was replaced by 0.184 g (0.2 mmol) of $HRh(CO)(PPh_3)_3$ and 0.262 g (1.0 mmol) of ($PPh_3$). The reaction product was analyzed by gas chromatography. The results are shown in the table below.

Example 4

The hydroformylation reaction was performed on 4-cyclohexene-1,2-dicarboxylic acid anhydride in the same manner as in Example 1, except that the catalyst was replaced by 0.158 g (0.2 mmol) of dichlorobis(triphenylphosphine)platinum $[(PPh_3)_2PtCl_2]$ and 0.226 g (1.0 mmol) of $SnCl_2.2H_2O$, at a reaction temperature of 100°C, under a reaction pressure of $5.88 \times 10^6$ Pa (60 kg/$cm^2$), for a reaction time of 4 hours. The reaction product was analyzed by gas chromatography. The results are shown in the table below, in which reaction pressures are shown in units of $10^6$Pa, with kg/$cm^2$ equivalents being given in brackets [].

TABLE

| Example | Catalyst (mmol) | Reaction Temperature (°C) | Reaction Pressure in $10^6$Pa [$kg/cm^2$] | Reaction Time (hour) | Conversion of raw Material (mol %) | Selectivity of Reaction Products (mol %) Aldehyde (1) | Product A (2) | Product B (3) |
|---|---|---|---|---|---|---|---|---|
| 1 | $HRh(CO)(PPh_3)_3$ (0.2) | 115 | 1.96 [20] | 2 | 89.6 | 81.6 | 12.4 | 5.7 |
| 2 | $HRh(CO)(PPh_3)_3/PPh_3$ (0.2) (2.0) | 115 | 1.96 [20] | 2 | 88.5 | 96.9 | 2.5 | 0.6 |
| 3 | $HRh(CO)(PPh_3)_3/PPh_3$ (0.2) (1.0) | 115 | 1.96 [20] | 2 | 95.4 | 96.2 | 3.1 | 0.7 |
| 4 | $(PPh_3)_2Cl_2/SnCl_2.2H_2O$ (0.2) (1.0) | 110 | 5.88 [60] | 4 | 81.7 | 77.5 | 15.3 | 7.2 |

*Note*: (1) 4-formylcyclohexane-1,2-dicarboxylic acid anhydride
(2) 1-cyclohexene-1,2-dicarboxylic acid anhydride
(3) cyclohexane-1,2-dicarboxylic acid anhydride

**Claims**

1. Compounds represented by the formula:

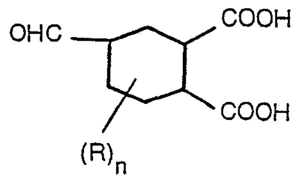

where R is an alkyl group, alkoxyl group, hydroxyl group or halogen atom, the R's being the same or different, and n is a number from 0 to 4, or a derivative thereof selected from the (mono, di)ester, the (mono, di)acid halide, the (mono, di)acid amide, the (mono, di)nitrile and the acid anhydride.

2. Compounds according to claim 1 in which the alkyl group has 1 to 4 carbon atoms and the alkoxyl group has 1 to 4 carbon atoms.

3. The compound 4-formylcyclohexane-1,2-dicarboxylic acid or its anhydride.

4. A process for preparing a compound represented by the formula:

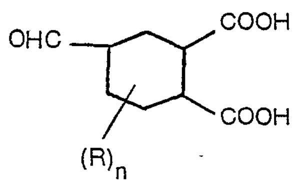

where R is an alkyl group, alkoxyl group, hydroxyl group or halogen atom, the R's being the same or different, and n is a number from 0 to 4, or a derivative thereof selected from the (mono, di)ester, the (mono, di)acid halide, the (mono, di)acid amide, the (mono, di)nitrile and the acid anhydride, said process comprising reacting a compound represented by the formula:

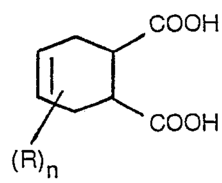

or the respective derivative thereof, where R and n are defined as above, with hydrogen and carbon monoxide in the presence of a hydroformylation catalyst.

5. A process according to claim 4 in which the alkyl group has 1 to 4 carbon atoms and the alkoxyl group has 1 to 4 carbon atoms.

6. A process according to claim 4 in which 4-formylcyclohexane-1,2-dicarboxylic acid anhydride is prepared from 4-cyclohexene-1,2-dicarboxylic acid anhydride.

7. A process according to claim 4, 5 or 6 in which the hydroformylation catalyst comprises catalysts containing rhodium, cobalt or platinum.

8. A process according to claim 7 in which the rhodium catalyst comprises rhodium carbonyl or a rhodium carbonyl complex compound with a ligand having the formula $Z(R^1)\ (R^2)\ (R^3)$ where Z denotes a phosphorus, arsenic or antimony atom and $R^1$, $R^2$, $R^3$ denote the same or different alkyl, alkoxyl, aryl or aryloxy groups.

9. A process according to claim 8 in which there is additionally present a ligand having the formula $Z(R^1)\ (R^2)\ (R^3)$.

10. A process according to claim 9 in which the rhodium catalyst is a rhodium carbonyl complex compound with a tertiary phosphine ligand and there is additionally present a tertiary phosphine ligand.

11. A process according to claim 9 in which the rhodium catalyst is $HRh(CO)(PPh_3)_3$ and the ligand present is $PPh_3$ in a ratio of 0.1 to 300 mols per mol of the rhodium catalyst.

12. A process according to claim 11 in which the ratio is 2 to 50 mols of ligand per mol of the rhodium catalyst.

13. A process according to any one of claims 4 to 12, wherein the hydroformylation catalyst comprises a catalyst containing rhodium and in which the reaction pressure is in the range of $9.8 \times 10^5$ to $2.94 \times 10^7$Pa (10 to 300 kg/cm$^2$), the temperature is in the range of 30° to 150°C, and the hydrogen/carbon monoxide molar ratio is in the range of 1/10 to 10/1.

**Patentansprüche**

1. Verbindungen, dargestellt durch die Formel:

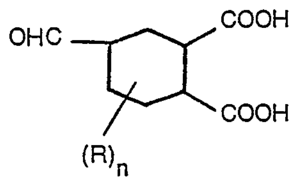

worin R eine Alkylgruppe, Alkoxylgruppe, Hydroxylgruppe oder ein Halogenatom bedeutet, wobei die Reste R gleich oder verschieden sind, und n eine Zahl von 0 bis 4 darstellt, oder ein Derivat davon ausgewählt aus dem (Mono-, Di-)ester, dem (Mono-, Di-)säurehalogenid, dem (Mono-, Di-)säureamid, dem (Mono-, Di-)nitril und dem Säureanhydrid.

2. Verbindungen nach Anspruch 1, worin die Alkylgruppe 1 bis 4 Kohlenstoffatome aufweist und die Alkoxylgruppe 1 bis 4 Kohlenstoffatome aufweist.

3. Die Verbindungen 4-Formylcyclohexan-1,2-dicarbonsäure oder deren Anhydrid.

4. Verfahren zur Herstellung einer Verbindung, die durch die Formel

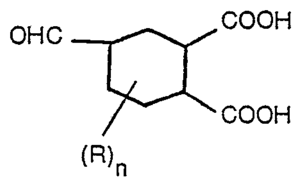

dargestellt wird, worin R eine Alkylgruppe, Alkoxylgruppe, Hydroxylgruppe oder ein Halogenatom darstellt, wobei die Reste R gleich oder verschieden sind, und n eine Zahl von 0 bis 4 darstellt, oder ein Derivat davon ausgewählt aus dem (Mono-, Di-)ester, dem (Mono-, Di-)säurehalogenid, dem (Mono-, Di-)säureamid, dem (Mono-, Di-)nitril und dem Säureanhydrid, wobei dieses Verfahren darin besteht, daß man eine Verbindung, die durch die Formel:

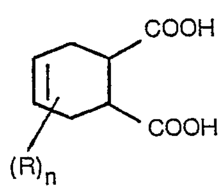

dargestellt wird, oder das entsprechende Derivat davon, worin R und n die vorstehende Bedeutung haben, mit Wasserstoff und Kohlenmonoxid in Gegenwart eines Hydroformylierungskatalysators umsetzt.

5. Verfahren gemäß Anspruch 4, worin die Alkylgruppe 1 bis 4 Kohlenstoffatome aufweist, und die Alkoxylgruppe 1 bis 4 Kohlenstoffatome aufweist.

6. Verfahren gemäß Anspruch 4, worin 4-Formylcyclohexan-1,2-dicarbonsäureanhydrid aus 4-Cyclohexen-1,2-dicarbonsäureanhydrid hergestellt wird.

7. Verfahren gemäß Anspruch 4, 5 oder 6, worin der Hydroformylierungskatalysator Rhodium, Kobalt oder Platin enthaltende Katalysatoren umfaßt.

8. Verfahren gemäß Anspruch 7, worin der Rhodiumkatalysator Rhodiumcarbonyl oder eine Rhodiumcarbonylkomplexverbindung mit einem Liganden mit der Formel $Z(R^1)$ $(R^2)$ $(R^3)$ umfaßt, worin Z ein Phosphor-, Arsen- oder Antimonatom bedeutet und $R^1$, $R^2$, $R^3$ die gleiche oder verschiedene Alkyl-, Alkoxyl-, Aryl- oder Aryloxygruppen bedeuten.

9. Verfahren gemäß Anspruch 8, worin zusätzlich ein Ligand mit der Formel $Z(R^1)$ $(R^2)$ $(R^3)$ vorhanden ist.

10. Verfahren gemäß Anspruch 9, worin der Rhodiumkatalysator eine Rhodiumcarbonyl-komplexverbindung mit einem tertiären Phosphinliganden ist und zusätzlich ein tertiärer Phosphinligand vorhanden ist.

11. Verfahren gemäß Anspruch 9, worin der Rhodiumkatalysator $HRh(CO)$ $(PPh_3)_3$ ist und der vorhandene Ligand $PPh_3$ in einem Verhältnis von 0,1 bis 300 Mol pro Mol des Rhodiumkatalysators ist.

12. Verfahren gemäß Anspruch 11, worin das Verhältnis 2 bis 50 Mol Ligand pro Mol des Rhodiumkatalysators ist.

13. Verfahren gemäß einem der Ansprüche 4 bis 12, worin der Hydroformylierungskatalysator einen Rhodium enthaltenden Katalysator umfaßt und wobei der Reaktionsdruck im Bereich von $9{,}8 \times 10^5$ bis $2{,}94 \times 10^7$ Pa (10 bis 300 kg/cm$^2$) liegt, die Temperatur im Bereich von 30 bis 150°C liegt und das Molverhältnis von Wasserstoff/Kohlenmonoxid im Bereich von 1/10 bis 10/1 liegt.

**Revendications**

1. Composés représentés par la formule:

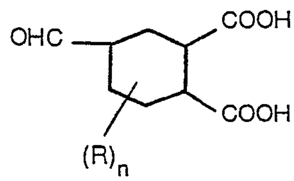

dans laquelle R est un groupe alkyle, un groupe alkoxy, un groupe hydroxyle ou un atome d'halogène, les groupes R étant égaux ou différents, en *n* est un nombre de 0 à 4, ou un dérivé de ces composés choisi entre le (mono, di)ester, le (mono, di)halogénure d'acide, le (mono, di)amide d'acide, le (mono, di)nitrile et l'anhydride d'acide.

2. Composés suivant la revendication 1, dans lesquels le groupe alkyle comprend 1 à 4 atomes de carbone et le groupe alkoxy comprend 1 à 4 atomes de carbone.

3. L'acide 4-formylcyclohexane-1,2-dicarboxylique ou son anhydride.

4. Procédé de préparation d'un composé représenté par la formule:

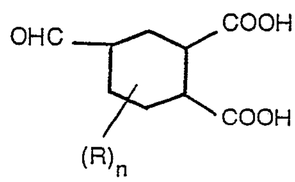

où R est un groupe alkyle, un groupe alkoxy, un groupe hydroxyle ou un atome d'halogène, les groupes R étant égaux ou différents, en *n* est un nombre de 0 à 4, ou d'un dérivé de ce composé choisi entre le (mono, di)ester, le (mono, di)halogénure d'acide, le (mono, di)amide d'acide, le (mono, di)nitrile et l'anhydride d'acide, procédé caractérisé en ce qu'il consiste à faire réagir un composé représenté par la formule:

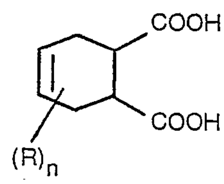

ou le dérivé respectif de ce composé, où R est *n* ont les définitions données ci-dessus, avec de l'hydrogène et de l'oxyde de carbone en présence d'un catalyseur d'hydroformylation.

5. Procédé suivant la revendication 4, dans lequel le groupe alkyle comprend 1 à 4 atomes de carbone et le groupe alkoxy comprend 1 à 4 atomes de carbone.

6. Procédé suivant la revendication 4, dans lequel l'anhydride d'acide 4-formylcyclohexane-1,2-dicarboxylique est préparé à partir d'anhydride d'acide 4-cyclohexene-1,2-dicarboxylique.

7. Procédé suivant la revendication 4, 5 ou 6, dans lequel le catalyseur d'hydroformylation comprend des catalyseurs contenant du rhodium, du cobalt ou du platine.

8. Procédé suivant la revendication 7, dans lequel le catalyseur au rhodium comprend du rhodium-carbonyle ou un composé complexe de rhodium-carbonyle avec un ligand de formule $Z(R^1)$ $(R^2)$ $R^3)$ dans laquelle Z désigne un atome de phosphore, d'arsenic ou d'antimoine et $R^1$, $R^2$, $R^3$ désignent des groupes alkyle, alkoxy, aryle ou aryloxy qui sont égaux ou différents.

9. Procédé suivant la revendication 8, dans lequel il y a en outre un ligand de formule $Z(R^1)$ $(R^2)$ $(R^3)$.

10. Procédé suivant la revendication 9, dans lequel le catalyseur au rhodium est un composé complexe de rhodium-carbonyle avec un ligand phosphinique tertiaire et un ligand phosphinique tertiaire est en outre présent.

11. Procédé suivant la revendication 9, dans lequel le catalyseur au rhodium a la formule $HRh(CO)(PPh_3)_3$ et le ligand présent est $PPh_3$, dans un rapport de 0,1 à 300 moles par mole de catalyseur au rhodium.

12. Procédé suivant la revendication 11, dans lequel le rapport est de 2 à 50 moles de ligand par mole de catalyseur au rhodium.

13. Procédé suivant l'une quelconque des revendications 4 à 12, dans lequel le catalyseur d'hydroformylation comprend un catalyseur contenant du rhodium et la pression de réaction se situe dans l'intervalle de $9,8 \times 10^5$ à $2,94 \times 10^7$ Pa (10 à 300 $kg/cm^2$), la température va de 30 à 150°C et le rapport molaire de l'hydrogène à l'oxyde de carbone se situe dans l'intervalle de 1/10 à 10/1.